# EUROPEAN PATENT SPECIFICATION

(11) **EP 3 629 754 B1**
(45) Date of publication and mention of the grant of the patent: **10.05.2023**
(21) Application number: 18756306.9
(22) Date of filing: 29.05.2018
(51) Int. Cl.: A23K 10/10, A23K 10/30, A23K 20/195, A23K 50/70, A23K 50/75, A61K 36/185, A23L 33/105

(54) **METHOD OF PREPARING ADROSERA**
VERFAHREN ZUR HERSTELLUNG EINES FUTTERMITTELZUSATZES AUF SONNTENTAUBASIS
MÉTHODE DE PRÉPARATION D'UN ADDITIF POUR ALIMENTATON ANIMALE À BASE DE DROSERA

(30) Priority: 29.05.2017 PL 42174717
(43) Date of publication of application: 08.04.2020
(73) Proprietor: Herbiopharm Sp. z.o.o., 80-180 Gdansk (PL)
(72) Inventor: BANASIUK, Rafa, 84-100 Puck (PL); WILCZYNSKI, Jaroslaw, 62-080 Lusowko (PL); NIDZWORSKI, Dawid, 80-180 Gdansk (PL)
(74) Representative: Matyka, Malgorzata
(86) International application number: PCT/PL2018/000054
(87) International publication number: WO 2018/222060

(56) References cited:
- WO-A1-2004/019961
- WO-A1-2014/081976
- CN-A- 106 234 825
- FR-A- 1 160 700
- FR-M- 1 230
- JP-A- 2004 000 081
- JP-A- 2006 347 995

## Description

The scope of this invention is a way for preparing a feed additive based on *in vitro* obtained *Drosera* plants. The additive has a positive influence on the microflora of *Gallus gallus domesticus* and other *Aves.*

*Drosera* plants (sundews) are a natural source of pharmacologically active compounds that are known for their anti-cough, diuretic, antimicrobial and anticancer properties. First known uses of sundews for pharmacological use was noted in the XII century. The plants were used for treating dry cough, bronchitis, whooping-cough and asthma. *Drosera* plant extracts were also found useful in treating urinary tract infections and skin discolourations caused by *Mycobacterium leprae.* Some reports even point to the use of sundews as headache remedies.

Sundews were found to be useful in medicine during the natural remedy epoch, before the epoch of synthetic compounds. They owe their activity to a broad range of secondary metabolites, that possess biological activity such as immunostimulatory (they stimulate the immune system to react to foreign microorganisms and infectious agents), antimicrobial, antispasmodic and antitumor.

Some sundews are found on the list of endangered species. Because of this, the plant material is obtained by *in vitro* plant culture micropropagation. It is an effective way of obtaining plant material and simultaneously plant secondary metabolites without any integration in the natural environment.

The essence of this invention is the use of *Drosera spp.* metabolites for creating a feed additive. The use of this invention in poultry production may lead to lowering the number of bacterial and fungal infections that would lead to reducing antibiotic usage.

The description WO2014081976A1 disclose plant material which is obtained from normal growth conditions that require washing and drying. These steps are omitted when using the method presented in our invention. Additionally, standardisation of normally grown plant material due to biological variety is practically impossible, while in vitro cultures use clones. WO2014081976A1 puts emphasis on the antiviral activity that is not the activity shown in our invention (bactericidal and fungicidal)

The description JP 2004000081 disclose invention which relates to a method for producing a vegetable extract, but the sundew is not a vegetable - it is classified as a medicinal herb since it is not consumed as a nutritional source. The invention JP 2004000081 provide a method for producing a vegetable extract having a more natural and desirable flavor that does not have the green odor, freshness, astringency, and taste of green vegetable juice.

The description FR1160700 presents to use a percolator for the extraction process and uses metionted plant extracts as the means to demonstrate the process efectivness. In our invention we do not use this kind of device. Additionally the concentration of alcohol used is insufficient for propper extraction of the compounds present in Drosera plant material. The process is fundamentally different from the one presented in our invention.

The another description FR1230M presents to a homeopatic tincture that does not possess even a single molecule of active substance in the final product. According to the description, the acrivity of the tincture comes from the serial dillution process when in our invention we do not claim such activity.

The description WO2004019961A1 disclosed a methodology for finding new potential anticancer substances. This application does not mention any results of Drosera extract activity. Addidtionally our invention concearns the antimicrobial activity of Drosera plant material produced in vitro. This is not the scope of the presented record.

The patent application description JP2006347995A presents the use of Drosera rotundifolia. The invention requires a 5 day extraction and the claims do not concern the biological activity whatsoever. Drosera rotundifolia does not present in the claims of the patent.

The present invention relates to a method of preparing a *Drosera sp.* based feed additive comprising of:
a. Cultivation of *Drosera sp.* plants in *vitro* cultures
b. Solvent extraction with ethanol by shaking or ultrasound-assisted.
c. Dilution of the extract with water to a content of 20 to 0.31 % ethanol.

The advantage of the use of *Drosera* plant secondary metabolites for the preparation of a feed additive is that it has a positive effect on the bird microflora. The invention uses only natural compounds without any synthetic additives.

### Table description:

**Tab.1** - Antimicrobial analysis of *Drosera sp.* extract was performed by a serial dilution method as follows: The analysis was performed in a 96-well plate. For each of the tested microorganism, a 0,5 McFarland suspension was prepared. Next 100µl of the prepared suspension was added to 10mL of Miller-Hinton broth. In the next step 100µl of the diluted microbial suspension was added. Next 100µl of plant extract was added that was diluted with the serial dilution method to obtain the effective concentration range. The results presented in the table concern the extract dilution where microbial growth inhibition was observed.

The invention is demonstrated by the following examples that do not limit the use of the invention.

### Example 1.

Preparation of *Drosera sp.* based feed additive from *in vitro* grown plants.
1. *Drosera* plant material was grown on ½ Murashige Skoog medium with the addition of 2% sucrose (pH 5,5) and cleaned from the remains of the medium.
2. 60% ethanol and water mix were prepared. 150 grams of plant material per litre was added.
3. Plant material was extracted for an hour with shaking.
4. The extract was diluted to 20% ethanol content and then was tested for antimicrobial properties.

The prepared extract was tested for antimicrobial activity. The results are presented in Table 1.

### Example 2.

Preparation of *Drosera sp.* based feed additive from *in vitro* grown plants.
1. *Drosera* plant material was grown on Murashige Skoog medium with the addition of 3% sucrose and cleaned from the remains of the medium.
2. 70% ethanol and water mix were prepared. 200 grams of plant material per litre was added.
3. Plant material was extracted for 15 minutes in an ultrasonic bath at room temperature.
4. The extract was diluted to 10% ethanol content and then was tested for antimicrobial properties.

The prepared extract was tested for antimicrobial activity. The results are presented in Table 1.

## Claims

1. A method of preparing a *Drosera sp.* based feed additive comprising of:
a. Cultivation of *Drosera sp.* plants in *vitro* cultures,
b. Solvent extraction with ethanol by shaking or ultrasound-assisted,
c. Dilution of the extract with water to a content of 20 to 0.31 % ethanol.
**Ta b. 1**
| No | Microorganism | Effective extract concentration | Microorganism carrier | Place of occurrence | Extract from Example: |
|---|---|---|---|---|---|
| 1 | *Pseudomonas aeruginosa* | 10% | broiler | Internal organs | 1 |
| 2 | *Enterococcus faecalis* | 5% | broiler | Internal organs | 1 |
| 3 | *Escherichia coli* | No effect | broiler | Internal organs | 1 |
| 4 | *Pseudomonas aeruginosa* | 10% | broiler | Internal organs | 1 |
| 5 | *Pasteurella multocida* | 2,50% | goose | Internal organs | 2 |
| 6 | *Gallibacterium anatis* | 1,25% | hen | Internal organs | 2 |
| 7 | *Enterococcus cecorum* | 2,50% | turkey | Internal organs | 2 |
| 8 | *Escherichia coli* | 10% | broiler | Internal organs | 2 |
| 9 | *Escherichia coli* | 10% | hen | Internal organs | 2 |
| 10 | *Escherichia coli* | 10% | hen | Internal organs | 1 |
| 11 | *Salmonella Enteritidis* | 10% | hen | Internal organs | 2 |
| 12 | *Enterococcus faecalis* | 5% | hen | Internal organs | 2 |
| 13 | *Staphylococcus saprophiticis* | 1,25% | broiler | Internal organs | 2 |
| 14 | *Salmonella Bethune* | 10% | | feathers | 1 |
| 15 | *Candida albicans* | 0,63% | turkey | intestines | 2 |
| 16 | *Candida albicans* | 0,63% | turkey | intestines | 1 |
| 17 | *Pseudomonas aeruginosa* | 10% | broiler | Internal organs | 1 |
| 18 | *Streptococcus alactolyticus* | 0,16% | pigeon | cloaca | 2 |
| 19 | *Enterococcus columbae* | 10% | pigeon | cloaca | 1 |
| 20 | *Enterococcus faecalis* | 5% | broiler | Internal organs | 2 |
| 21 | *Candida albicans* | 1,25% | turkey | intestines | 2 |
| 22 | *Candida rugosa* | 0,31% | broiler | intestines | 2 |
| 23 | *Candida catenulata* | 1,25% | broiler | intestines | 2 |

## Patentansprüche

1. Der Herstellungsprozess eines Futtermittelzusatzes aus Drosera sp. umfasst:
a. Kultivierung von Pflanzenmaterial der Gattung Drosera sp. über In-vitro-Kulturen,
b. Lösungsmittelextraktion mit Ethanol durch Schütteln oder mit Hilfe von Ultraschall,
c. Verdünnung des Extraktes mit Wasser bis zu einem Gehalt von 20 bis 0.31 % Ethanol.

## Revendications

1. Procédé de préparation d'un additif alimentaire à partir de Drosera sp., comprenant :
a. Cultures de matériel végétal de l'espèce Drosera sp. en cultures *in vitro,*
b. Extractions au solvant d'éthanol par agitation ou par ultrasons,
c. Dilution de l'extrait avec de l'eau jusqu'à une teneur de 20 à 0,31 % d'éthanol.
